# EUROPÄISCHE PATENTSCHRIFT

(11) **EP 0 842 649 B1**
(45) Veröffentlichungstag und Bekanntmachung des Hinweises auf die Patenterteilung: **07.01.2004**
(21) Anmeldenummer: 97117869.4
(22) Anmeldetag: 15.10.1997
(51) Int. Cl.: A61F 13/10

(54) **Selbstklebende Fertigbandage für Ellenbogen**
Adhesive bandage for the elbow
Bandage adhésif pour le coude

(30) Priorität: 13.11.1996 DE 19646741
(43) Veröffentlichungstag der Anmeldung: 20.05.1998
(73) Patentinhaber: Beiersdorf Aktiengesellschaft, 20245 Hamburg (DE)
(72) Erfinder: Bodenschatz, Stefan, Dr., 21614 Buxtehude (DE); Himmelsbach, Peter, 21614 Buxtehude (DE)

(56) Entgegenhaltungen:
- DE-A- 3 843 483
- DE-A- 4 312 655
- DE-C- 19 512 013
- US-A- 3 888 244
- US-A- 3 989 041

## Beschreibung

Die Erfindung betrifft eine einseitig selbstklebend beschichtete Fertigbandage zur Stützung und Funktionseinschränkung des Ellenbogengelenks. Die dadurch bewirkte Entlastung des Gelenks dient insbesondere zur Behandlung von Extensionstraumen, Reizzuständen z.B. des Kapsel-Band-Apparates, Überlastungsbeschwerden des M. biceps brachii, M. brachialis, M. brachioradialis oder Epikondylitis radialis.

Die funktionelle Verbandtechnik, das sog. Taping, ist eine gängige Behandlungsmethode zur Prophylaxe und Therapie von Verletzungen, Erkrankungen und Veränderungen am Bewegungsapparat. Taping hat zum Ziel, die einzelnen Weichteile und Kapsel-, Band-Strukturen gezielt nachzubilden bzw. deren Funktionen selektiv zu stützen.

Der Tapeverband wird aus mehreren Bändern, sog. Zügeln, streifenweise unter Verwendung von vorzugsweise unelastischem Material, teilweise kombiniert mit elastischem Material, angelegt und erfüllt dann die Funktionen Stützen und Entlasten.

Derartige Verbände erfordern jedoch fachmännisches Können und sehr viel Erfahrung und können deshalb in aller Regel nicht von im Taping Unerfahrenen angelegt werden.

Aus der DE-C 195 12 013 ist eine das Anlegen erleichternde selbstklebende Fertigbandage zur Stützung und partiellen Fixierung des Ellenbogengelenks bekannt, die aus einem länglichen Streifen besteht, der einseitig in Längsrichtung bis etwa zur Mitte des Streifens einen Einschnitt aufweist. Eine derartige Bandage eignet sich jedoch nicht für alle medizinisch gegebenen Indikationen, insbesondere ist sie nicht für eine Einschränkung der Extension des Gelenks konzipiert.

DE 4312655 A1 beschreibt eine Sprunggelenkbandage, die eine Längsrichtung und eine Querrichtung hat, bestehend aus einem im wesentlichen rechteckigen, in Querrichtung der Bandage mindestens teilweise unelastischen Teil A, an welchem in Längsrichtung gesehen nach oben und unten gerichtet jeweils zwei längliche Streifen B und C sowie D und E angeordnet sind.

Zum Unterschied umfasst die erfindungsgemäße Bandage, dass der Streifen A 8 cm lang und 12 cm breit ist, der Teil A wenigstens zu einem Teil in Querrichtung eine Höchstzugkraft-Dehnung von weniger als 30% aufweist und die Streifen B, C, D und E 35 cm lang und 6 cm breit sind.

Aufgabe der Erfindung war es deshalb, eine Fertigbandage zu entwickeln, die aufgrund ihrer Ausgestaltung das Ellenbogengelenk stützt, entlastet und eine Einschränkung der Extension des Gelenkes ermöglicht.

Gelöst wird diese Aufgabe durch eine Bandage gemäß Anspruch 1.

Der Mittelteil A ist, in Querrichtung der Bandage gesehen, vorzugsweise unelastisch ausgebildet, er kann jedoch auch nur teilweise unelastisch sein. Zum Beispiel in der Weise, daß das Material in dieser Richtung elastisch oder dehnbar ist, jedoch unelastische Verstärkungsstreifen aufweist. Der Teil A soll im angelegten Zustand, wie später noch gezeigt wird, im wesentlichen in Längsrichtung des Armes verlaufen. Die Querrichtung ist also die Wirkungsrichtung dieses Teiles der Bandage.

Der Mittelteil A besitzt als Ganzes bzw. zumindest teilweise in Querrichtung der Bandage eine Höchstzugkraft-Dehnung von weniger als 30%.

Die Streifen B, C, D und E können in einem Winkel ∝ von 30 bis 150°, bezogen auf die Querrichtung der Bandage, an dem Teil A angeordnet sein, wobei alle Variationen in der Ausrichtung der jeweiligen Streifen möglich sind. Vorzugsweise stehen sie jedoch senkrecht auf dem Mittelteil A, d.h. der Winkel ∝ beträgt 90° und die vier langen Streifen verlaufen parallel zueinander.

Die Länge der Streifen ist durch deren Anlegetechnik, die weiter unten noch genauer beschrieben wird, vorgegeben. Beispielhaft beträgt die Länge der Bandage insgesamt 80 cm und sie ist 12 cm breit. Dabei ist das Mittelteil A 8 cm lang und 12 cm breit und die Streifen B, C, D und E sind 35 cm lang und 6 cm breit Je nach Größenverhältnis des zu bandagierenden Armes können sie auch länger und breiter sein oder gegebenenfalls gekürzt werden. Weiterhin können die Streifen (Zügel) unterschiedlich ausgebildet sein, d.h. beispielsweise können sie in der Dicke, Breite, Länge oder den textiltechnischen Kenndaten variieren.

Die Bandage ist insgesamt einstückig ausgebildet, wobei sie entweder als Ganzes aus einem größeren Stück Bandagenmaterial ausgeschnitten, ausgestanzt oder aus Einzelteilen zusammengefügt wird.

Sie besteht besonders bevorzugt aus einem in einer Richtung, d.h. der späteren Längsrichtung der Bandage, dehnbaren oder elastischen textilen Material, vorzugsweise aus einem in dieser Richtung dehnbaren oder elastischen Gewebe oder Gewirke insbesondere auf Baumwollbasis mit einer Höchstzugkraft von vorzugsweise mindestens 60 N/cm und einer Dehnfähigkeit bei einer Belastung von 10N/cm bis zu etwa 90%, bevorzugt 10% bis 80%, in Längsrichtung. In der anderen Richtung, d.h. quer dazu, sollte die Höchstzugkraft-Dehnung maximal 30% betragen.

Das Material ist dabei in der Bandage so ausgerichtet, daß das Mittelteil A in Querrichtung mindestens teilweise unelastisch ist und die Streifen B, C, D und E in Längsrichtung dehnbar oder elastisch sind. In der Querrichtung sind diese Streifen bevorzugt unelastisch. Sie können jedoch auch in dieser Richtung Elastizität bzw. Dehnbarkeit aufweisen.

Als sehr günstig hat es sich erwiesen, wenn sich am inneren Ende des Einschnitts zwischen den Streifen D und E, d.h. am Schnittpunkt von A, D und E, eine Ausnehmung in Form eines Dreiecks befindet. Dadurch wird der Sitz der Bandage verbessert und das Anlegen erleichtert. Das Dreieck ist in der Weise angebracht, daß seine Basis entlang A quer zur Längsrichtung der Bandage verläuft und seine Spitze, nach unten zeigend, das Ende des Einschnitts zwischen D und E darstellt. Es ist vorzugsweise gleichschenklig und in etwa auch gleichseitig. Seine Fläche beträgt etwa 12 cm² und seine Ecken können abgerundet sein. Es sind jedoch auch andere Aussparungsformen möglich. Es ist auch möglich, auf die Aussparung ganz zu verzichten oder nur eine kleine Ausnehmung anzubringen.

Am inneren Ende des Einschnitts zwischen den Streifen B und C befindet sich vorteilhaft ebenfalls eine kleine Ausnehmung, um ein Einreißen zu verhindern.

Auf ihrer der Haut zugewandten Seite ist die Bandage mit einer der bekannten gut haftenden Selbstklebemassen beschichtet auf Basis von Kautschuk oder synthetischen Polymeren. Vorzugsweise sollte diese luft- und wasserdampfdurchlässig sowie gut hautverträglich sein.

Bis zum Gebrauch der Bandage kann die Klebeschicht mit einem klebstoffabweisend ausgerüsteten Blattmaterial, wie beispielsweise silikonisiertem Papier oder Kunststoff-Folie, abgedeckt sein.

Es hat sich dabei als günstig erwiesen, diese Abdeckung mehrteilig, vorzugsweise 5-teilig, durch z.B. perforierte Trennlinien auszubilden. Dabei deckt ein Teil das Mittelteil A ab und 4 weitere streifenförmige Teile die schmaleren Streifen B, C, D und E. Die Abdeckungsteile können als Anbringungshilfe farbig markiert oder nummeriert sein.

Beim Anlegen der Bandage am angewinkelten Arm wird zuerst der Mittelteil A auf der lateralen Gelenkseite fixiert. Dieses geschieht so, daß der Mittelteil A der Breite nach - also in Querrichtung - die aus Unter- und Oberarm gebildeten Schenkel gleichwinklig schneidet und sich über das gesamte Ellenbogengelenk erstreckt. Die dreieckige Ausnehmung soll dabei so ausgerichtet sein, daß der Epikondylus frei bleibt. Dies erleichtert die Anpassung der Bandage beim Anlegen. Dann wird der Streifen B von medial nach lateral zirkulär um den Oberarm geführt und angeklebt, so daß er zumindest einen Teil, bevorzugt den ganzen Oberarm wenigstens einmal umschließt. Die Streifen B, C, D, E werden als Zügel bezeichnet. Als nächster Zügel wird der Streifen C angelegt. Dieser verläuft von medial nach lateral am Unterarm, so daß er zumindest einen Teil, bevorzugt den ganzen Unterarm und gegebenenfalls auch noch den Handrücken wenigstens einmal umschließt. Bei beiden Zügeln B und C ist darauf zu achten, daß der innere Beugebereich des Ellenbogens nicht verklebt wird.

Dann wird der Zügel D angebracht. Er verläuft unterhalb des Ellenbogens distal zum Unterarm und wird von dort medial zirkulär nach lateral geführt. Er soll zumindest einen Teil, bevorzugt den ganzen Unterarm wenigstens einmal umschließen.

Zuletzt wird der Zügel E angelegt, indem er proximal vom Unterarm zum Oberarm und dort von medial kommend zirkulär nach lateral geführt wird.

Die klebstoffabweisenden Abdeckmaterialien auf den Streifen werden entsprechend dem Vorgehen beim Ankleben der Reihe nach entfernt. Die Bandage kann zusätzlich durch Ankerstreifen aus üblichem Tapematerial verstärkt werden.

Die fertig angelegte Bandage stützt, fixiert und entlastet das Ellenbogengelenk, wobei die Einschränkung der Gelenkfunktion durch den in Querrichtung wenigstens teilweise unelastischen Streifen A gewährleistet wird, die Streifen B und C fixierend und unterstützend wirken, der Streifen D das Strecken des Unterarms einschränkt und der Streifen E den Epikondylus entlastet. Außerdem weist die Bandage eine propiorezeptive Wirkung auf und unterstützt damit die mechanische Einschränkung der Gelenkfunktion.

Ein vereinfachtes Anlegen der Fertigbandage ist jedoch auch möglich. Hierbei werden die Zügel D und E nur zirkulär um den Unter- bzw. Oberarm angelegt. Durch ein Überlappen der Zügel BID und C/E wird die Fixierung des Mittelteils A auch bei starker Belastung gewährleistet. Besonders einfach läßt sich dieses Überlappen durch eine Dreiecksform der Abdeckung auf der selbstklebenden Seite des Mittelteils A erreichen. Hierbei können die Zügel B und C noch unter dem Mittelteil A geführt werden. Dieses verstärkt die Fixierung.

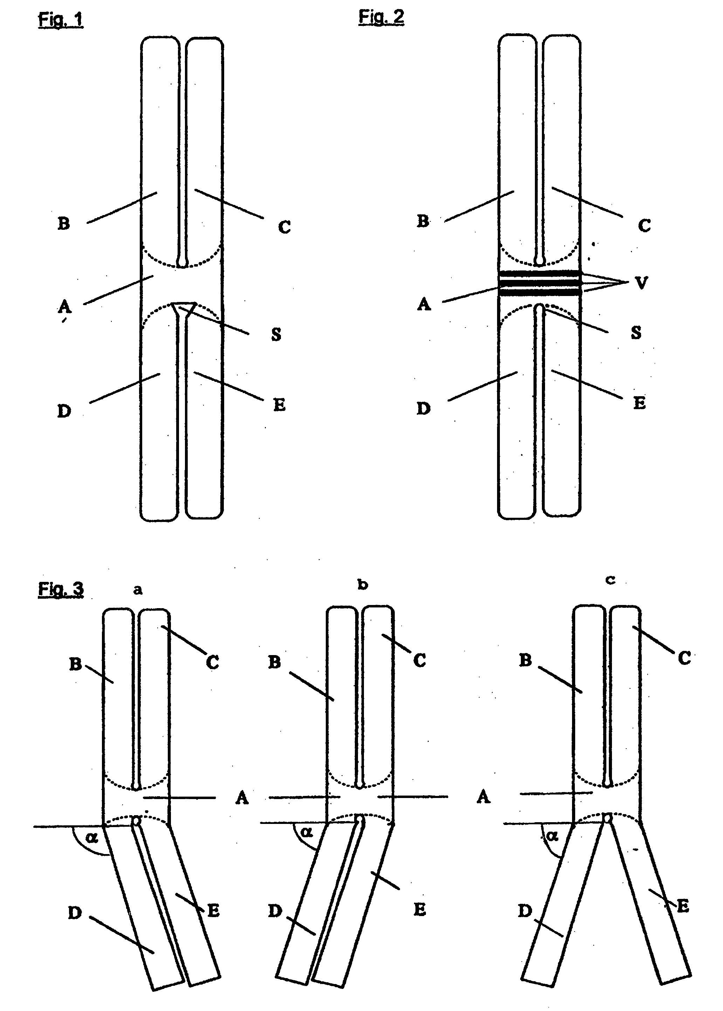

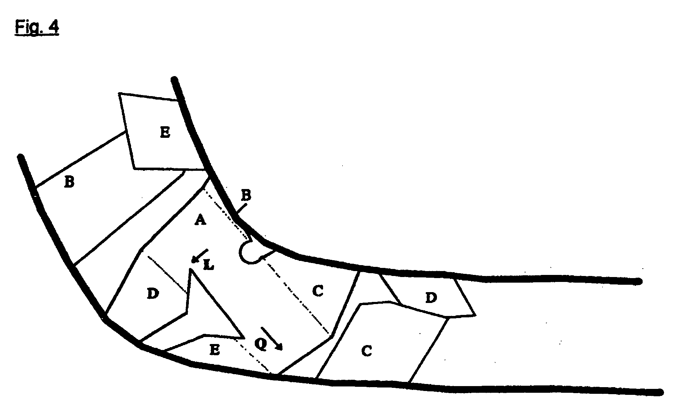

Die erfindungsgemäße Bandage ist in Figur 1 beispielsweise dargestellt, wobei die länglichen Streifen (Zügel) B, C, D und E senkrecht zu dem Mittelteil A angeordnet sind. D.h., der Winkel ∝ beträgt 90°. Mit S ist der Schnittpunkt von A, D und E bezeichnet Die gestrichelten Linien bedeuten die Perforation in der Abdeckung auf der klebenden Seite der Bandage.

Figur 2 zeigt beispielhaft eine längs- und querelastische Bandage mit drei unelastischen Verstärkungsstreifen (V), die im Mittelteil A in Querrichtung mit der Bandage verbunden sind. Der Mittelteil A ist dadurch in Querrichtung teilweise unelastisch und damit erfindungsgemäß wirksam.

Figur 3 zeigt unter a, b und c drei Variationen der Anordnung der Zügel B, C, D und E mit einem Winkel ∝ der Zügel D und E zur Querrichtung der Bandage von jeweils ca. 115° bei a und jeweils ca. 65° bei b sowie ca. 65° für D und ca. 115° für E bei c.

Figur 4 zeigt die Bandage im angelegten Zustand mit einer Wickelung wie weiter oben beschrieben.

## Patentansprüche

1. Auf einer Seite selbstklebend beschichtete Bandage zur Entlastung und Funktionseinschränkung des Ellenbogengelenks, die eine Längsrichtung und eine Querrichtung hat, bestehend aus einem rechteckigen, in Querrichtung der Bandage mindestens teilweise unelastischen Teil A, an weichem in Längsrichtung gesehen nach oben und unten gerichtet jeweils zwei längliche Streifen B und C sowie D und E angeordnet sind, **dadurch gekennzeichnet, dass** der Streifen A 8 cm lang und 12 cm breit ist, der Teil A wenigstens zu einem Teil in Querrichtung eine Höchstzugkraft-Dehnung von weniger als 30% aufweist und die Streifen B, C, D und E 35 cm lang und 6 cm breit sind.

2. Bandage nach Anspruch 1, **dadurch gekennzeichnet, dass** der Streifen B, C, D und E in einem Winkel ∝ von 30 bis 150° bezogen auf die Querrichtung der Bandage an dem Teil A angeordnet sind.

3. Bandage nach Anspruch 1 oder 2, **dadurch gekennzeichnet, dass** der Winkel ∝ 90° beträgt.

4. Bandage nach einem der vorhergehenden Ansprüche, **dadurch gekennzeichnet, dass** die Streifen B, C, D und E in Längsrichtung dehnbar oder elastisch sind.

5. Bandage nach einem der vorhergehenden Ansprüche, **dadurch gekennzeichnet, dass** sich am inneren Schnittpunkt S von A, D und E zwischen diesen Teilen eine Ausnehmung befindet in Form eines Dreiecks, dessen Basis entlang A quer zur Längsrichtung der Bandage verläuft.

6. Bandage nach einem der vorhergehenden Ansprüche, **dadurch gekennzeichnet, dass** sie auf ihrer klebstoffbeschichteten Seite mit einem klebstoffabweisenden Material abgedeckt ist.

7. Bandage nach Anspruch 6, **dadurch gekennzeichnet, dass** das klebstoffabweisende Trägermaterial 5-teilig ausgebildet ist, wobei je ein Teil die Streifen A, B , C, D und E abdeckt.

## Claims

1. Bandage with a self-adhesive coating applied on one side, which bandage is intended to relieve and to restrict the function of the elbow joint, has a longitudinal direction and a transverse direction and consists of a rectangular part A which at least in part is nonelastic in the transverse direction of the bandage and on which there are arranged, viewed in the longitudinal direction, two elongate strips B, C and D, E directed upwards and downwards respectively, **characterized in that** the strip A is 8 cm long and 12 cm wide, the part A has at least in part in the transverse direction an ultimate tensile strength elongation of less than 30%, and the strips B, C, D and E are 35 cm long and 6 cm wide.

2. Bandage according to Claim 1, **characterized in that** the strips B, C, D and E are arranged on the part A at an angle α of 30° to 150° relative to the transverse direction of the bandage.

3. Bandage according to Claim 1 or 2, **characterized in that** the angle α is 90°.

4. Bandage according to one of the preceding claims, **characterized in that** the strips B, C, D and E are extensible or elastic in the longitudinal direction.

5. Bandage according to one of the preceding claims, **characterized in that**, at the inner intersection S of A, D and E, a recess is situated between these parts, said recess being in the form of a triangle whose base extends along A transversely with respect to the longitudinal direction of the bandage.

6. Bandage according to one of the preceding claims, **characterized in that** it is covered on its adhesive-coated side with an anti-adhesive material.

7. Bandage according to Claim 6, **characterized in that** the anti-adhesive support material is formed in 5 parts, one part each covering strips A, B, C, D and E.

## Revendications

1. Bandage pourvu d'adhésif d'un seul côté pour décharger et limiter le fonctionnement de l'articulation du coude, qui présente une direction longitudinale et une direction transversale, se composant d'une partie A rectangulaire au moins partiellement non élastique dans la direction transversale du bandage, sur laquelle sont disposées, vues dans la direction longitudinale, deux bandes respectives longitudinales B et C et D et E orientées vers le haut et vers le bas, **caractérisé en ce que** la bande A a une longueur de 8 cm et une largeur de 12 cm, la partie A présentant au moins en partie, dans la direction transversale, une extension maximale sous tension inférieure à 30% et les bandes B, C, D et E mesurant 35 cm de long et 6 cm de large.

2. Bandage selon la revendication 1, **caractérisé en ce que** les bandes B, C, D et E sont disposées suivant un angle α de 30 à 150° par rapport à la direction transversale du bandage au niveau de la partie A.

3. Bandage selon la revendication 1 ou 2, **caractérisé en ce que** l'angle α vaut 90°.

4. Bandage selon l'une quelconque des revendications précédentes, **caractérisé en ce que** les bandes B, C, D et E sont extensibles ou élastiques dans la direction longitudinale.

5. Bandage selon l'une quelconque des revendications précédentes, **caractérisé en ce qu'**un évidement de forme triangulaire, dont la base s'étend le long de A transversalement à la direction longitudinale du bandage, se trouve au niveau du point d'intersection interne S de A, D et E entre ces pièces.

6. Bandage selon l'une quelconque des revendications précédentes, **caractérisé en ce qu'**il est recouvert sur son côté enduit d'adhésif d'un matériau antiadhésif.

7. Bandage selon la revendication 6, **caractérisé en ce que** le matériau de support antiadhésif est réalisé en 5 parties, chaque partie recouvrant respectivement les bandes A, B, C, D et E.
